(19) Europäisches Patentamt / European Patent Office / Office européen des brevets

(11) **EP 2 753 918 B1**

(12) **EUROPEAN PATENT SPECIFICATION**

(45) Date of publication and mention
of the grant of the patent:
**17.07.2019 Bulletin 2019/29**

(21) Application number: **12770332.0**

(22) Date of filing: **04.09.2012**

(51) Int Cl.:
*A61B 5/00* (2006.01)     *A61B 5/1459* (2006.01)
*A61B 5/145* (2006.01)     *A61B 5/1455* (2006.01)
*A61B 5/1473* (2006.01)    *G01N 21/64* (2006.01)
*G01N 21/77* (2006.01)     *G01N 33/66* (2006.01)

(86) International application number:
**PCT/US2012/053704**

(87) International publication number:
**WO 2013/036492 (14.03.2013 Gazette 2013/11)**

(54) **OPTICAL GLUCOSE SENSOR**

OPTISCHER GLUCOSESENSOR

CAPTEUR DE GLUCOSE OPTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 06.09.2011  US 201161531449 P
06.09.2011  US 201161531451 P
06.09.2011  US 201161531456 P
01.11.2011  US 201161554057 P
17.11.2011  US 201161561146 P
18.01.2012  US 201261587819 P
05.04.2012  US 201261620563 P
23.05.2012  US 201213478478

(43) Date of publication of application:
**16.07.2014 Bulletin 2014/29**

(73) Proprietor: **Medtronic MiniMed, Inc.**
**Northridge, CA 91325-1219 (US)**

(72) Inventors:
• **AASMUL, Soren**
**DK-2840 Holte (DK)**
• **KRISTENSEN, Jesper, Svenning**
**DK-2830 Virum (DK)**
• **EJLERSEN, Henning, Munk**
**DK-2950 Vedbaek (DK)**

(74) Representative: **Ruschke, Hans Edvard**
**Ruschke Madgwick Seide & Kollegen**
**Postfach 86 06 29**
**81633 München (DE)**

(56) References cited:
WO-A1-2006/061207     WO-A1-2011/075575
US-A1- 2003 171 666     US-A1- 2005 113 658

• **Ralph Ballerstadt ET AL: "Fiber-Coupled Fluorescence Affinity Sensor for 3-Day in Vivo Glucose Sensing", J Diabetes Sci Technol, vol. 1, no. 3 May 2007 (2007-05), pages 384-393, XP055044981, Retrieved from the Internet: URL:http://www.ncbi.nlm.nih.gov/pmc/articl es/PMC2769588/pdf/dst-01-0384.pdf [retrieved on 2012-11-20]**
• **JANNIK K NIELSEN ET AL: "Clinical Evaluation of a Transcutaneous Interrogated Fluorescence Lifetime-Based Microsensor for Continuous Glucose Reading", JOURNAL OF DIABETES SCIENCE AND TECHNOLOGY, vol. 3, no. 1, 1 January 2009 (2009-01-01) , pages 98-109, XP055042790,**
• **TANAKA K ET AL: "COMPOUND PARABOLIC CONCENTRATOR PROBE FOR EFFICIENT LIGHT COLLECTION IN SPECTROSCOPY OF BIOLOGICAL TISSUE", APPLIED OPTICS, OPTICAL SOCIETY OF AMERICA, WASHINGTON, DC; US, vol. 35, no. 4, 1 February 1996 (1996-02-01), pages 758-763, XP000577616, ISSN: 0003-6935, DOI: 10.1364/AO.35.000758**
• **NIKOLAY A. DENISOV: "<title>Comparison of competing fiber optic probes for tissue fluorescence analysis</title>", PROCEEDINGS OF SPIE, vol. 4161, 16 November 2000 (2000-11-16), pages 234-243, XP055051756, ISSN: 0277-786X, DOI: 10.1117/12.409334**

**Description**

## FIELD OF THE INVENTION

Embodiments of the present invention relate generally to sensor technology, including sensors used for sensing a variety of physiological parameters, e.g., glucose concentration. More particularly, embodiments of the invention relate to optical sensors, to methods of making and using such sensors, to optical and optoelectronic systems for interrogating optical sensors, and to methods of making and using such optical/optoelectronic systems. More particularly still, embodiments of the invention relate to optical fiber sensors including a fluorophore-labeled assay, to stacked planar optical integrated systems for interrogating such optical fiber sensors, and to methods of making and using such optical fiber sensors and optical integrated systems.

## BACKGROUND

Epifluorescence microscopy is a method of fluorescence microscopy that is becoming increasingly used in the biological and medical fields. An epifluorescence microscope is used primarily to excite a specimen by passing a source light through an objective lens and then onto the specimen. The fluorescence in the specimen generates emitted (fluorescent) light which is focused onto a detector by the same objective lens that is used for the excitation. Since most of the source light is generally transmitted through the specimen, only reflected source light reaches the objective lens together with the fluorescent light. An additional filter between the objective lens and the detector can filter out the remaining source light from fluorescent light.

The underlying principles of epifluorescence microscopy may be used in optical, or optoelectrical, systems for interrogating assay-based glucose sensors. The assay in such sensors may be interrogated using a variety of methods, such as Streak Camera recording, single photon counting, frequency domain lifetime measurement, and steady state fluorescence measurement. In both the frequency domain lifetime and steady state fluorescence interrogation, the function of the optical interrogation system is to excite the assay fluorophore(s) and prevent the excitation light from reaching the detector(s) while, at the same time, transmitting the emitted fluorescence. It is understood that the fluorescence emitted from fluorophore-labeled assays is generally weak. Therefore, it is important to excite the assay as efficiently as possible and to gather as much of the isotropically emitted fluorescence as possible.

In the context of a continuous glucose monitor based on frequency domain lifetime interrogation and steady state fluorescence interrogation, it is important not only to minimize the cost, size, and weight of the (optical system) instrumentation and of the optical sensor, but also to optimize manufacturability of both the instrumentation and the sensor. In this regard, the currently-used optical systems are in general fairly large and expensive, and require precision assembly as they include a number of different optical components. Thus, improved optical systems and optical glucose sensors, including sensors for use with such optical systems, are needed that address the above-mentioned requirements.

The publication "Fiber-Coupled Fluorescence Affinity Sensor for 3-Day in Vivo Glucose Sensing" by R. Ballerstadt et al., Journal of Diabetes Science and Technology, Volume 1, Issue 3, May 2007, pages 384-393 describes an optical glucose sensor as defined in the preamble of claim 1, wherein the assay comprises Concanavalin A labeled with AF750 as glucose receptor and dextran labeled with AF647 as glucose analog. A similar fiber optic glucose sensor is disclosed in US 2003/171666 A. In this publication Concanavalin A labeled with TRITC is used as glucose receptor and dextran labeled with FITC is used as glucose analog. In both cases a ratio of measured fluorescence intensities of the donor and acceptor fluorophore is calculated as an output indicative of glucose concentration.

The publication "Clinical Evaluation of a Transcutaneous Interrogated Fluorescence Lifetime-Based Microsensor for Continuous Glucose Reading", by J. K. Nielsen et al., Journal of Diabetes Science and Technology, Volume 3, Issue 1, January 2009, pages 98-109 describes a glucose sensor based on fluorescence lifetime measurements, wherein the assay comprises human mannan-binding lectin (MBL) labeled with AF594 as glucose receptor and dextran labeled with hexamethoxy crystalviolet-1 (HMCV1) as glucose analog.

## SUMMARY

The present invention is directed to an optical glucose sensor as defined in claim 1. Preferred embodiments are defined in the dependent claims.

Features and advantages of the invention will become apparent from the following detailed description, taken in conjunction with the accompanying drawings which illustrate, by way of example, various features of embodiments of the invention.

**BRIEF DESCRIPTION OF THE DRAWINGS**

FIG. 1 shows a glucose binding competitive affinity assay based on Forster Resonance Energy Transfer (FRET);

FIG. 2 shows instrumentation for measuring the lifetime of the excited state for a fluorophore-labeled assay;

FIG. 3 shows a glucose binding competitive affinity assay, including a reference fluorophore, for intensity interrogation used in an optical glucose sensor according to the invention;

FIG. 4A shows instrumentation for interrogating a fluorophore-labeled assay with an internal reference used for an intensity interrogation;

FIG. 4B shows instrumentation for interrogating a fluorophore-labeled assay with an internal reference used for an intensity interrogation;

FIG. 5A is a perspective view of an optical fiber sensor used in an optical glucose sensor according to the invention;

FIG. 5B is a side view of the optical fiber sensor shown in FIG. 5A;

FIG. 6A is a perspective view of an optical fiber sensor with a compound parabolic concentrator (CPC)-shaped fiber tip;

FIG. 6B is a side view of the optical fiber sensor shown in FIG. 6A;

FIG. 7A is a perspective view of an optical fiber sensor with a compound parabolic concentrator (CPC)-shaped fiber tip, a reflector, and support structure;

FIG. 7B is a side view of the optical fiber sensor shown in FIG. 7A;

FIG. 7C is a side view of an optical fiber with side cut cavities;

FIG. 7D is a perspective view of the optical fiber sensor shown in FIG. 7C;

FIG. 8 shows the fluorescence distribution inside the assay compartment of an optical fiber sensor with a straight-cut optical fiber (height = diameter of assay compartment);

FIG. 9 shows the fluorescence distribution inside the assay compartment of an optical fiber sensor with a CPC-shaped optical fiber (height = diameter of assay compartment);

FIG. 10A shows the fluorescence distribution inside the assay compartment of an optical fiber sensor with a CPC-shaped optical fiber and reflector (height = diameter of assay compartment);

FIG. 10B shows the fluorescence distribution inside the assay compartments of an optical fiber sensor with side cut cavities (height = diameter of fiber);

FIG. 11 shows a stacked planar integrated optical system (SPIOS) for lifetime interrogation of a fluorophore-labeled assay;

FIG. 12 shows the spectrum of light source, excitation filter, and fluorophore for a lifetime system;

FIG. 13 shows a stacked planar integrated optical system (SPIOS) for intensity interrogation of a fluorophore-labeled assay;

FIG. 14 shows the spectrum of light source, excitation filter, emission filter, assay fluorophore, and reference fluorophore for a intensity system;

FIG. 15 shows a stacked planar integrated optical system (SPIOS) for intensity interrogation of a fluorophore-labeled assay;

FIGS. 16A and 16B show examples of a CPC SPIOS-fiber interface; and

FIG. 17 shows illustrative layers of a wafer-scale stacked planar integrated optical system (SPIOS).

## DETAILED DESCRIPTION

As shown in the drawings for purposes of illustration, the present disclosure is directed to optical sensors that may be interrogated by optical, or optoelectronic, systems. Optical sensors may be introduced and/or lodged transdermally, or may be implanted in and/or through subcutaneous, dermal, sub-dermal, inter-peritoneal, or peritoneal tissue. In the discussion herein, preferred devices, systems, and methods of the disclosure are described with reference to glucose as the analyte whose level/concentration in the blood and/or bodily fluids of the user is to be determined. However, this is by way of illustration and not limitation, as the principles, devices, systems, and methods of the present disclosure may be used for sensing and/or determining the level of a variety of other physiological parameters, agents, characteristics, and/or compositions.

As will be described in more detail below, an optical glucose sensor having an assay compartment may be formed, e.g., by including a glucose permeable membrane containing the assay at the distal end of an optical fiber. The optical fiber may then be inserted transdermally into the user's body, thereby situating the assay compartment in the user's tissue, while leaving at least a part of the optical fiber outside the body such that it can be accessed by an interrogating system. Alternatively, the optical sensor may be implantable, e.g., as part of an implantable glucose monitor including an interrogating optoelectronic system and a power source. The assay compartment may be formed between a glucose permeable membrane and an optical interface to the optoelectronic system. The optical sensor may preferably be biodegradable.

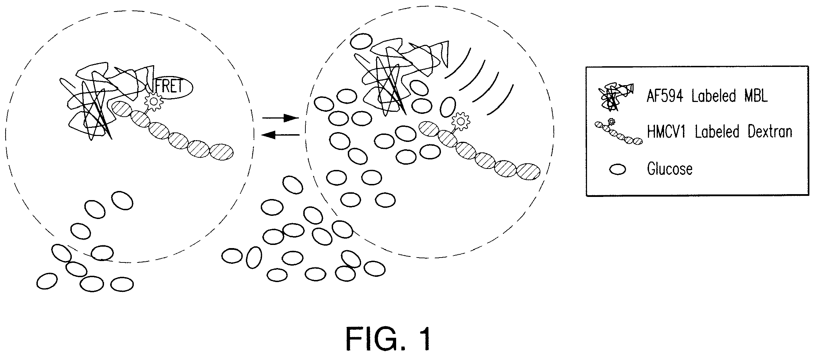

As shown in Figure 1, an optical glucose sensor may be based on a competitive glucose binding affinity assay. The assay may include a glucose receptor and a glucose analog (ligand) contained in a compartment where at least a part of the compartment is capable of exchanging small molecules, such as glucose, salts, etc. with the surrounding medium, while retaining macromolecules, such as the assay components.

Several molecules may serve as the glucose receptor in the glucose assay. Examples include, but are not limited to, Concanavalin A, periplasmic glucose/galactose-binding receptor, antibodies raised against glucose-like molecules, Boronic Acids, and Mannan Binding Lectin (MBL). Mannan Binding Lectin is human protein, which is a part of the innate immune system. Thus, the assay may include MBL as the glucose receptor and dextran as the glucose analog.

The binding between MBL and glucose-like molecules (e.g., dextran) is reversible. When no glucose is present, MBL and dextran will predominantly be bound together. When glucose is added to the assay, it will compete off a part of the dextran population, such that the assay enters a new equilibrium state. The equilibrium state at all times corresponds to the glucose concentration. In order to determine this equilibrium state, MBL is labeled with a fluorophore (e.g., Alexa Fluor 594, or AF594), and the dextran is labeled with a dye (e.g., hexamethoxy crystalviolet-1 (HMCV1) - a proprietary crystal violet derivative, Medtronic, Inc.). The donor fluorophore and the acceptor dye together form a Forster Resonance Energy Transfer (FRET) pair -- i.e., the emission spectrum of the fluorophore and the absorption spectrum of the dye overlap.

The occurrence of FRET affects the lifetime of the excited state and the intensity of the emitted fluorescence and can only occur when the fluorophore and the corresponding dye are in close proximity (i.e., in the range of about 50Å). Thus, the FRET mechanism permits interrogation of the equilibrium state optically by illuminating the assay and measuring either the lifetime of the excited state, and/or the intensity of the emitted fluorescence from the donor fluorophore. It is noted that the donor fluorophore and the acceptor dye are preferably water soluble, as they are to function in an aqueous environment.

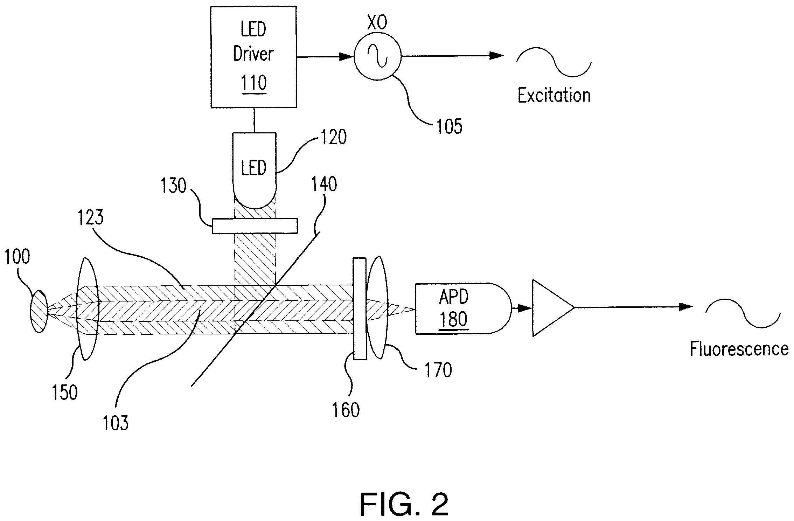

Figure 2 shows instrumentation used for frequency domain lifetime interrogation of the above-described assay based on a modified epifluorescence microscope. The instrumentation, or optical interrogation system, is optically coupled to (or aligned with) a sensor 100 carrying the assay. The assay is excited with a periodic signal (e.g., sinusoidal, squarewave, dirac pulse, approximative dirac, sawtooth, etc.), and the modulation frequency is governed by the lifetime of the excited state ($\tau$) for the fluorophore. The optimum modulation frequency may be approximated by:

$$f_{opt} = 1/(2 * \pi * \tau) \hspace{3cm} \text{Eq. (1)}$$

Thus, for a lifetime of 3ns, e.g., the optimum modulation frequency ($f_{opt}$) is in the range of about 50 MHz to about 60 MHz.

With reference to Figure 2, an oscillator 105 in combination with a driver circuit 110 modulates a LED 120 with a wavelength range capable of exciting the fluorophore. The LED 120 output is filtered using a multilayer dielectrical filter 130 to select a distinct wavelength region. The filtered LED output is reflected by a dichroic beam splitter 140 and focused onto the sensor 100 (which contains the assay) by a lens 150. The assay emits fluorescence with the same frequency as the excitation (modulated LED output) and phase shifted as a result of the lifetime of the excited state for the fluorophore.

The emitted fluorescence 103 and the reflected excitation light 123 are picked up and collimated by the lens 150. The dichroic beam splitter 140 transmits the fluorescence 103. However, it reflects the majority of the back-reflected excitation light 123. An emission filter 160 with a distinct wavelength region red shifted with respect to, and not overlapping, the pass band of the excitation filter blocks the remaining part of the excitation light 123 and transmits the fluorescence 103. Thus, in effect, only the fluorescence carrying the modulated and phase shifted fluorescence is focused onto a photodetector 180 using a lens 170. The phase lag between the detected fluorescence and the excitation light correlates with the glucose concentration in the assay.

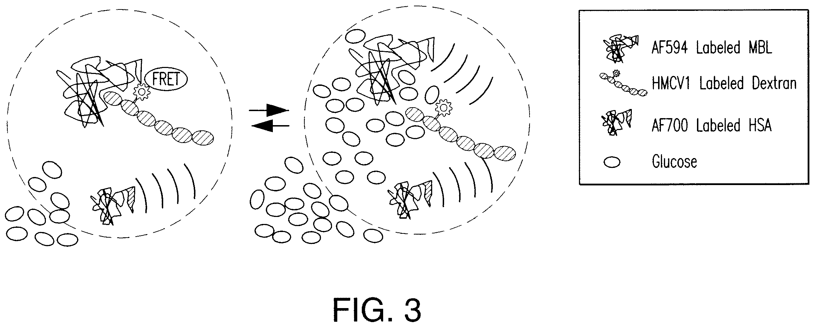

In addition to the lifetime of the excited state, the intensity of the emitted fluorescence also correlates to the glucose concentration. In contrast to a lifetime measurement, the measured intensity of the emitted fluorescence is affected by the intensity of the light source and the coupling between the assay and the optical system. Therefore, the intensity measurement requires an internal reference fluorophore to be incorporated into the assay, as shown in Figure 3.

The reference fluorophore must differ from the assay fluorophore in a way that the emitted fluorescence from the assay and that from the reference may be separated from one another, e.g., by having different absorption spectra or emission spectra. The reference fluorophore may be, e.g., Alexa Fluor 700 (AF700) labeled onto Human Serum Albumin (HSA) or another macro molecule, which largely does not bind to the glucose receptor. See Figure 3. Alexa Fluor 700 may be excited simultaneously with the Alexa Fluor 594 as their absorption spectra spectrally overlap. The emission spectrum from Alexa Fluor 700 is slightly red shifted with respect to Alexa Fluor 594, which makes it possible to detect their respective fluorescence emissions in separate wavelength regions. As they are excited simultaneously by the same light source, any changes in the intensity of the light source will scale fluorescence from AF594 and AF700 equally. As such, any effect originating from changes in the intensity of the light source may be cancelled out.

The excitation, as well as the detection, of the emitted fluorescence for the assay and the reference follow the same optical path from the optical system to the assay. As such, the detected signal from the reference serves as a measure for the optical coupling between the optical interrogating system and the assay. Any effect originating from changes in the optical coupling such as alignment may be cancelled out.

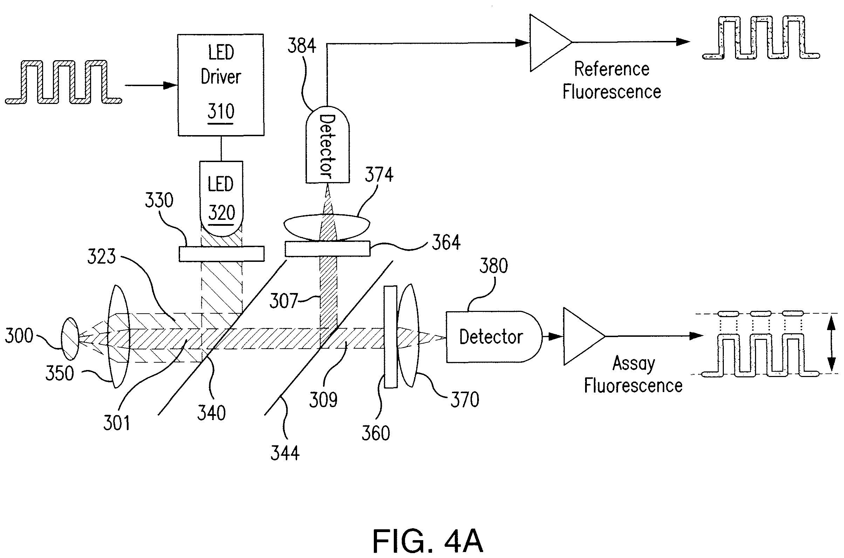

Figure 4A shows one example of instrumentation used for fluorescence interrogation of the above-described assay based on another modification of an epifluorescence microscope. A driver circuit 310 modulates a LED 320 at a low frequency--solely with the purpose of eliminating the 1/f noise and canceling out ambient light--with a wavelength range capable of simultaneously exciting the assay and reference fluorophores. The LED output is filtered using a multilayer dielectrical filter 330 to select a distinct wavelength region. The filtered LED output is reflected by a first dichroic beam splitter 340 and focused onto the sensor 300, which includes the assay and the reference, by a lens 350.

The assay and the reference emit fluorescence. The emitted fluorescence 301 and the reflected excitation light 323 are picked up and collimated by the lens 350. The first dichroic beam splitter 340 transmits the fluorescence 301. However, it reflects the majority of the back reflected excitation light 323. A second beam splitter 344 reflects the reference fluorescence at a 90° angle 307, but it transmits the assay fluorescence 309. An assay emission filter 360 with a distinct wavelength region red shifted with respect to, and not overlapping, the pass band of the excitation filter and matching the desired part of the assay fluorescence spectrum then blocks the remaining part of the excitation light and transmits the assay fluorescence.

Similarly, a reference emission filter 364 with a distinct wavelength region red shifted with respect to, and not overlapping, the pass band of the excitation filter and matching the desired part of the reference fluorescence blocks the remaining part of the excitation light and transmits the reference fluorescence 307. Thus, in effect, only the fluorescence from the assay and the fluorescence from the reference are focused onto their respective photo detectors 380, 384 using respective lenses 370, 374. The ratio between the detected assay fluorescence and the detected reference fluorescence correlates with the glucose concentration in the assay. As mentioned previously, any changes in light-source intensity or optical coupling will be cancelled out as they scale the assay and reference fluorescence equally.

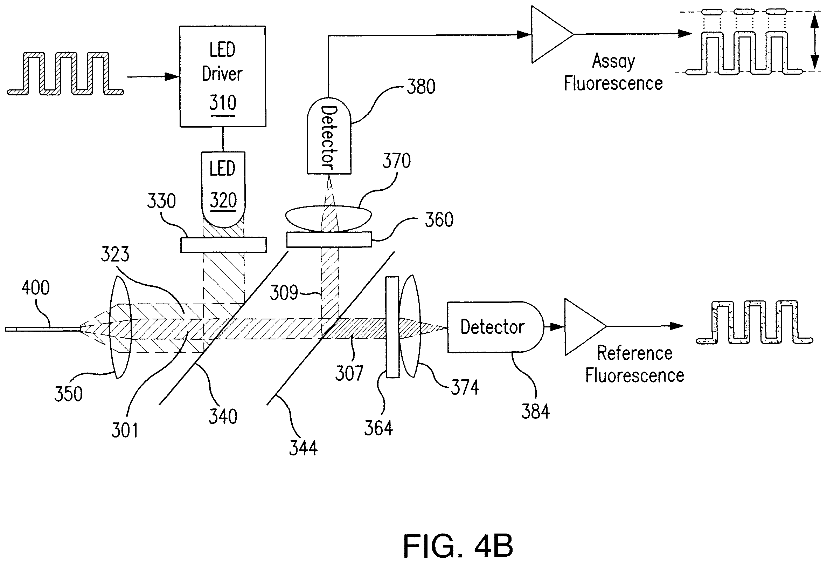

Figure 4B shows another example of the instrumentation used for fluorescence interrogation. Here, as in Figure 4A, driver circuit 310 modulates a LED 320 at a low frequency--solely with the purpose of eliminating the 1/f noise and canceling out ambient light--with a wavelength range capable of simultaneously exciting the assay and reference fluorophores. The LED output is filtered using a multilayer dielectrical filter 330 to select a distinct wavelength region. The filtered LED output is reflected by a first dichroic beam splitter 340 and focused onto the sensor 400, which includes the assay and the reference, by a lens 350. The sensor 400 is a fiber optical sensor, as described more fully hereinbelow.

As described in connection with Figure 4A, the assay and the reference emit fluorescence. The emitted fluorescence 301 and the reflected excitation light 323 are picked up and collimated by the lens 350. The first dichroic beam splitter 340 transmits the fluorescence 301. However, it reflects the majority of the back reflected excitation light 323. A second beam splitter 344 reflects the assay fluorescence at a 90° angle 309, but it transmits the reference fluorescence 307. A reference emission filter 364 with a distinct wavelength region red shifted with respect to, and not overlapping, the pass band of the excitation filter and matching the desired part of the reference fluorescence spectrum then blocks the remaining part of the excitation light and transmits the reference fluorescence.

Similarly, an assay emission filter 360 with a distinct wavelength region red shifted with respect to, and not overlapping, the pass band of the excitation filter and matching the desired part of the assay fluorescence blocks the remaining part of the excitation light and transmits the assay fluorescence 309. Thus, in effect, only the fluorescence from the assay and the fluorescence from the reference are focused onto their respective photo detectors 380, 384 using respective lenses 370, 374. The ratio between the detected assay fluorescence and the detected reference fluorescence correlates with the glucose concentration in the assay. Again, as mentioned previously, any changes in light-source intensity or optical coupling will be cancelled out as they scale the assay and reference fluorescence equally.

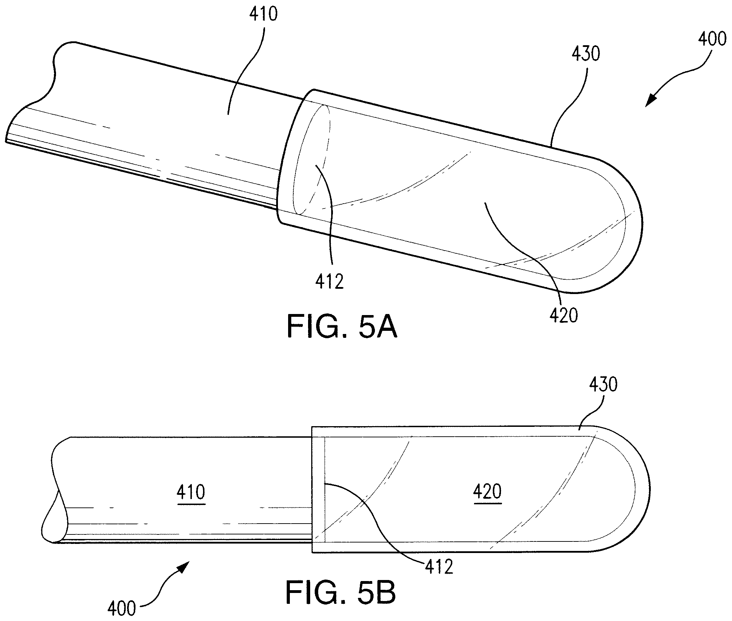

Figures 5A and 5B show an embodiment of the invention, wherein a fiber optical sensor 400 is made by placing the assay in a compartment 420 that is distal to the distal end 412 of an optical fiber 410. In this embodiment, a test tube-shaped glucose permeable membrane 430 containing the assay is slid over the end of the optical fiber 410 and sealed (e.g., heat sealed). The distal end 412 of the fiber 410 is straight cut and polished, and is in direct contact with the assay. In embodiments of the invention, the glucose permeable membrane 430 may be made of a biocompatible, biodegradable polymer such as, e.g., PolyActive™ (Integra Orthobiologics, Irvine, CA), Poly-lactide-glycolic-acid, poly-caaprolactone and non-biodegradable polymers exhibiting molecular weight cut-off properties like Cellulose (Spectrum Laboratories, Rancho Dominguez, CA) or Polysulfone (Spectrum Laboratories, Rancho Dominguez, CA).

With the above configuration, the assay may now be excited through the optical fiber 410, and the resulting fluorescence collected by the optical fiber. As the fluorescence from the assay radiates isotropically, the amount of the emitted fluorescence, which can be picked up by the optical fiber 410, is set by the numerical aperture of the fiber.

The numerical aperture (NA) of the optical fiber is a function of the refractive index of the fiber core ($n_1$) and the refractive index of the cladding ($n_2$):

$$NA = \sqrt{n_1^2 - n_2^2} \qquad\qquad \text{Eq. (2)}$$

Generally, light entering the optical fiber at an angle less than a critical angle will be transmitted through the optical fiber due to total internal reflection in the core/cladding boundary, whereas light entering at an angle larger than the critical angle will simply exit the fiber through the cladding. Commercially available optical fibers have a high refractive index core and a low refractive index cladding. Typical refractive indices for the core and the cladding for a plastic optical fiber are about 1.49 and about 1.40, respectively, which, based on Eq. (2), results in a numerical aperture of about 0.51. Per Eq. (3) below, this corresponds to a critical angle ($\theta$) of about 30.6°, or a solid angle of about 0.88sr:

$$NA = n \sin \theta_{max} \qquad\qquad \text{Eq. (3)}$$

For the ideal case, this translates into about a 7% pickup of the total emitted fluorescence--where isotropic radiation is $4\pi$sr, and 0.88sr/$4\pi$sr $\approx$ 7%. The maximum fluorescence pickup is thus set by the optical fiber.

Furthermore, some of the excitation light will spill out of the assay compartment through the glucose permeable membrane as the excitation light is coupled into the assay at angles corresponding to the fiber numerical aperture.

The Optical Invariant theorem states that the product of the source area (A) multiplied by the solid angle ($\Omega$) is constant:

$$A_1\Omega_1 = A_2\Omega_2 \qquad\qquad \text{Eq. (4)}$$

In Eq. (4), A1 is equal to the cross sectional area of the optical fiber, $\Omega$1 equals the solid angle corresponding to the numerical aperture of the optical fiber, and A2 and $\Omega$2 are set according to a trade-off between the maximum fiber tip length, saturation intensity for the assay, and manufacturable tip geometry.

Applied to the fiber sensor 400, this means that, with the right optical component and fiber tip design, the excitation light transmitted through the optical fiber 410 may be focused down to a smaller area moving the excitation away from the glucose permeable membrane 430 and thereby reduce the spillage of excitation light out of the sensor. In addition, the numerical aperture of the fiber tip may be increased resulting in an increased fluorescence pickup from the assay.

The Compound Parabolic Concentrator (CPC) is a non-imaging component, which has an entrance aperture, a parabolic shaped reflective surface, and an exit aperture. The CPC may be formed as an air filled void or an optical material with a parabolic mirror surface, or it may be formed by an optical material with a refractive index which is higher than that of the surrounding material. The parabolic shaped part of the CPC is formed as a parabola which ensures total internal reflection due to the high-low refractive index transition.

The radial coordinate of points on the CPC as a function of the z coordinate along the axis is given by the positive real root of the following quadratic equation:

$$C^2r^2 + 2(CSz + aP^2)r + (z^2S^2 - 2aCQz - a^2PT) = 0 \qquad \text{Eq. (5)}$$

Where C=cosθ, S=sinθ, P=1+S, Q=1+P, and T=1+Q

Shaping the tip of the optical fiber 410 as a CPC with the right dimensions will lead to the desired properties. As an example, a CPC shape applied to the tip of a 250μm optical fiber reduces tip diameter to 125μm. Area is thus reduced four times, leading to a theoretical four-fold increase in numerical aperture, which corresponds to a four-fold increase in fluorescence pickup.

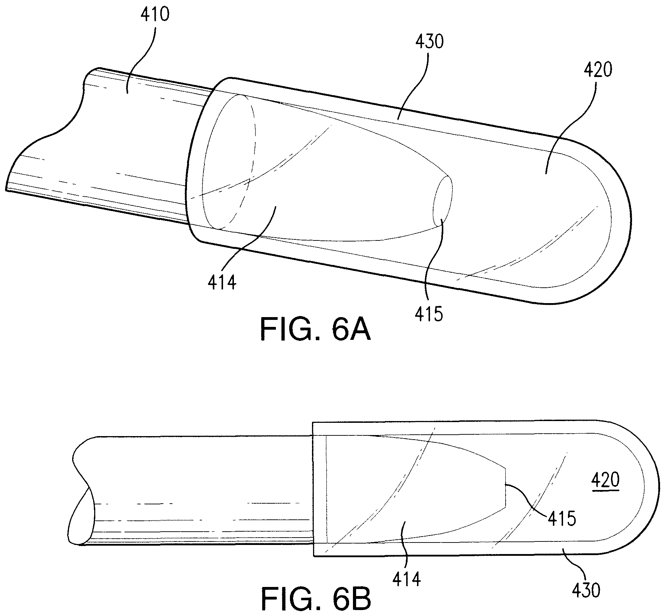

A fiber sensor employing a CPC tip geometry is shown in Figures 6A and 6B. As shown, the CPC shaped fiber tip 414 is in direct contact with the assay, which has a refractive index similar to water (1.33). As this is significantly lower than the refractive index of the optical fiber cladding, a CPC designed to have a cladding on the parabolic part will work with, as well as without, cladding.

It is noted that the theoretical four-fold increase in fluorescence pickup is based on the assumption that the fluorophore(s) of the assay are excited at the tip-assay transition, i.e., the CPC-assay optical interface 415. However, since the fluorescence occurs in a volume in front of the fiber tip 414, the increase in fluorescence pickup may be significantly less than the four-fold increase predicted by crude theoretical calculations.

Thus, even though, theoretically, the numerical aperture of the CPC fiber tip 414 will be increased dramatically compared to the conventional straight cut fiber tip 412, in operation, the CPC design generally cannot pick up more than about 50% of the emitted fluorescence. Nevertheless, the fluorescence pick-up percentage may be increased by placing a concave mirror 417 in front of the fiber tip 414 to reflect fluorescence emitted in a direction that is opposite to the fiber tip and focus it into the fiber.

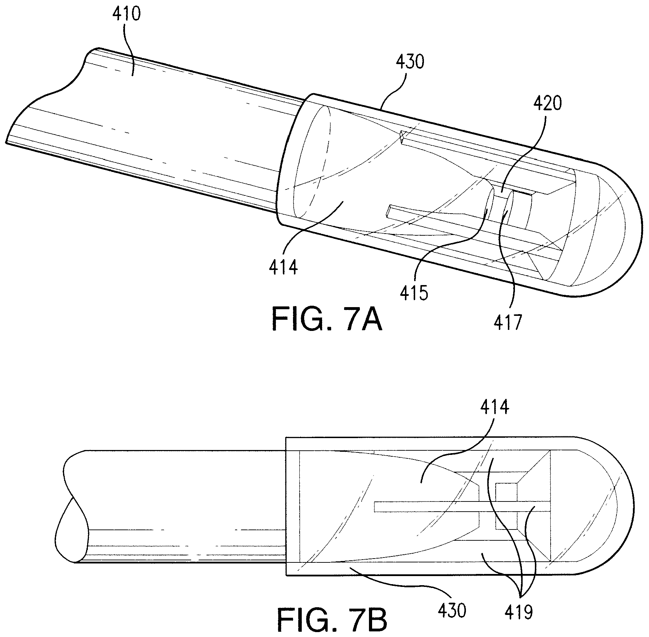

As shown in Figures 7A and 7B, there must still be a gap between the fiber tip 414 and the mirror 417 to leave room for the assay, i.e., to constitute the assay compartment 420. The mirror 417 obviously needs to be kept in place in front of the fiber tip 414. This may be accomplished, e.g., by the support structure 419, which allows glucose to diffuse into the space in front of the fiber tip 414. At the same time, the support structure 419 serves the purpose of supporting the glucose permeable membrane 430.

The straight cut fiber tip 412, the CPC shaped fiber tip 414, and the CPC shaped fiber tip in combination with a reflector 414, 417 have been modeled using Zemax optical design software. For each of the three designs, the model included the following: (1) an excitation light source at the proximal (free) end of the fiber sensor, coupling light into the optical fiber and exciting the assay at the (distal, in-situ) tip of the optical fiber; (2) an optical fiber with the selected fiber tip geometry; (3) an assay-filled compartment including assay absorption and assay fluorescence processes; and (4) a detector with a fluorescence filter only selecting the fluorescence picked up and transmitted back through the optical fiber.

For all three designs, the ratio between the excitation and the detected fluorescence was calculated. As can be seen from the results shown in Table 1 below, the Zemax simulations shows significantly lower fluorescence pickup from the CPC designs than the Optical Invariant theorem predicts. As stated, this is due to the fact that the excitation and resulting fluorescence emission occur in a volume in front of the fiber tip rather than directly at the fiber tip/assay boundary.

| TABLE 1 | | |
|---|---|---|
| | Without Reflector | With Reflector |
| Straight Cut | 100% | N/A |
| CPC | 166% | 277% |

In fiber sensors employing the CPC tip geometry described above, the assay, including the reference dye, can be carried or contained by a hydrogel in order to ease production and stabilize the assay. Specifically, the glucose assay is first dissolved in a hydrogel. Next, the CPC-shaped fiber tip may be dipped into the hydrogel containing the assay, and a droplet may be left in front of the CPC-shaped fiber tip. Finally, the hydrogel may be cross-linked so as to provide a glucose sensor. Suitable cross-linking hydrogels may include, e.g., Poly acryl based hydrogels (such as poly-Hydroxy-Ethyl-Methacrylate (pHEMA), PMMA-pHEMA co-polymers, etc.), Polyurethanes, Polyesters, Polyethers, etc.

Where the hydrogel containing the assay is not cross-linked, the entire arrangement could be coated by any of the polymers suitable for glucose sensors, i.e., polymers that allow glucose diffusion through the polymer. Suitable non-crosslinking hydrogels may include, e.g., poly-vinyl alcohol (PVA), Poly-ethylene glycol (PEG), poly-propylene glycol (PPG), poly-Hydroxy-Ethyl-Methacrylate (pHEMA), etc., and co-polymers thereof.

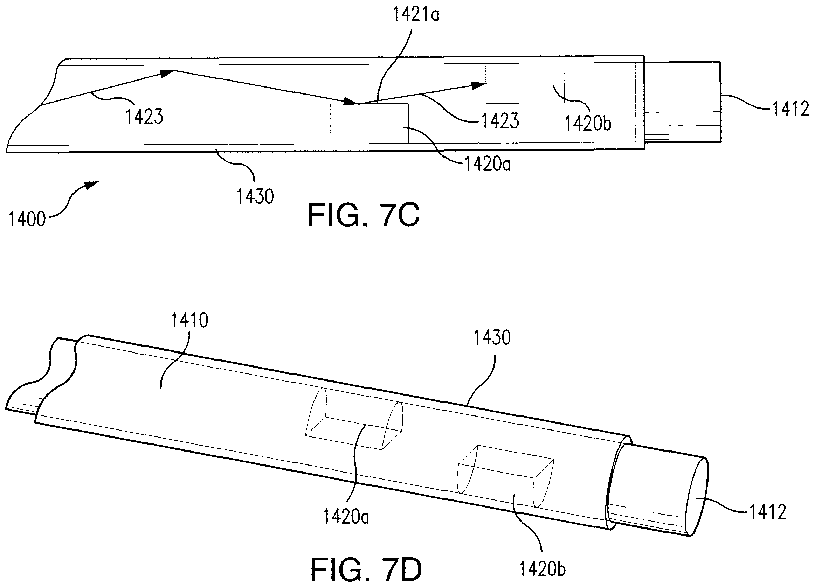

Figures 7C and 7D show another fiber optical sensor 1400 made by placing the assay in the compartments 1420a, 1420b proximate the distal end 1412 of an optical fiber 1410. The compartments are formed as side cuts in the fiber cutting through the fiber cladding as well as core. In this embodiment, a tube-shaped glucose permeable membrane 1430 is slid over the side cuts and sealed to the cladding of the fiber leaving glucose permeability in the regions of the assay compartments.

Two cuts are made on opposite sides of the fiber and displaced from each other sufficiently to maintain the structural strength of the fiber. For the first side cut cavity 1420a, the surface 1421a parallel to the fiber axis is preferably optical quality. As the refractive index of the assay is significantly lower than the fiber core, this will provide total internal reflection for excitation light 1423 traveling from the proximal end of the fiber sensor to the second assay compartment 1420b and furthermore provide total internal reflection for fluorescence emitted from the second assay compartment 1420b and back to the proximal end of the fiber. The above-described configuration provides structural strength to the fiber sensor, which is advantageous, especially for fiber sensors where a soft glucose permeable membrane in itself does not provide sufficient structural strength to ensure the stability of the assay compartment.

It is noted that fewer or more assay compartments may be included. For example, with reference to Figures 7C and 7D, the second assay compartment 1420b may be omitted when, e.g., a single assay compartment provides sufficient fluorescence. Also, in additional arrangements, as an alternative to the combination of a liquid assay and a glucose permeable membrane, a hydrogel with embedded assay as described above may be cast into the one or more side cavities to form the assay compartment(s).

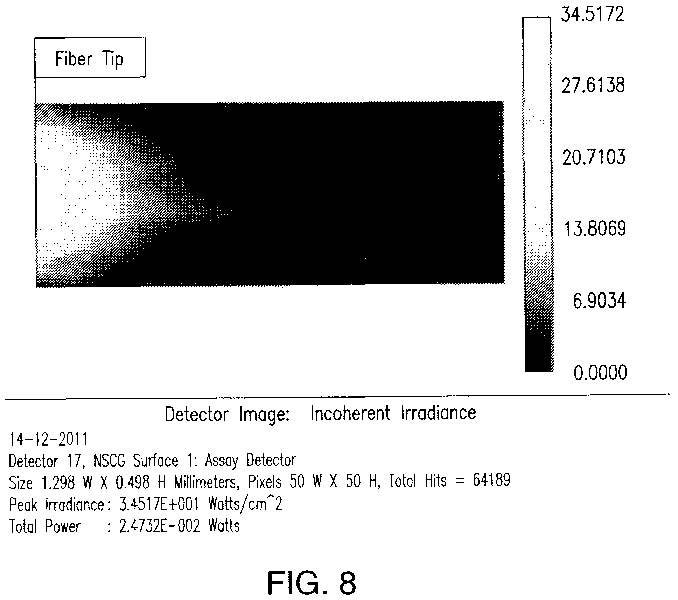

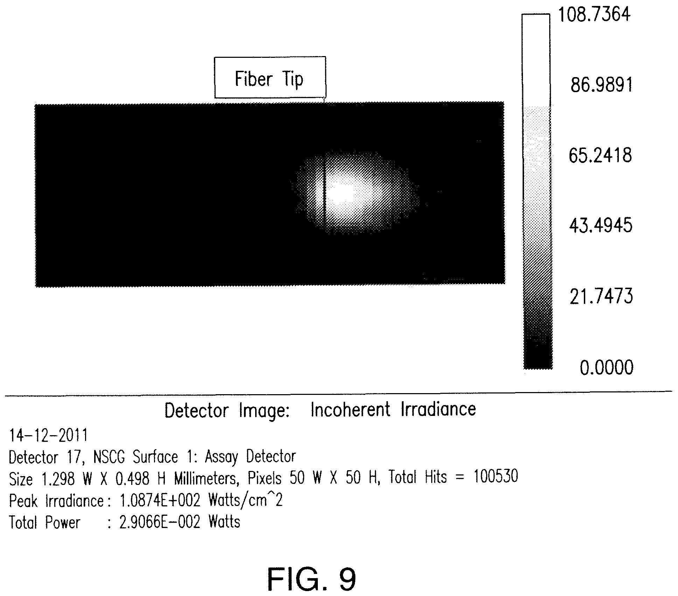

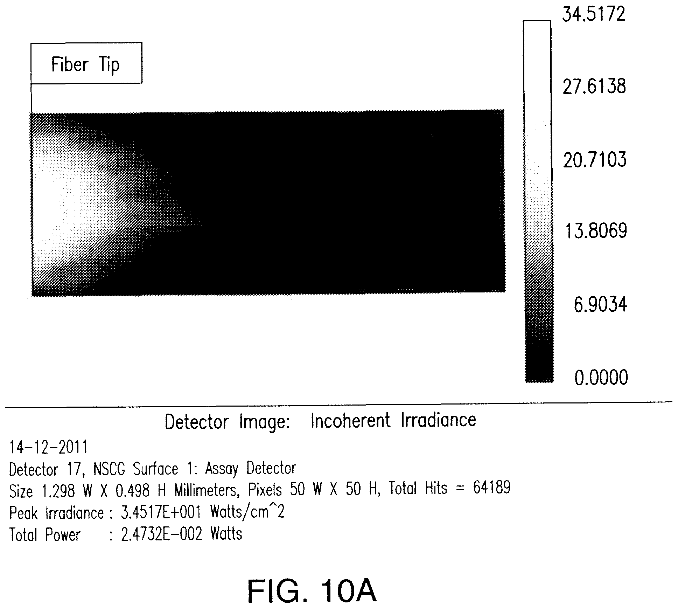

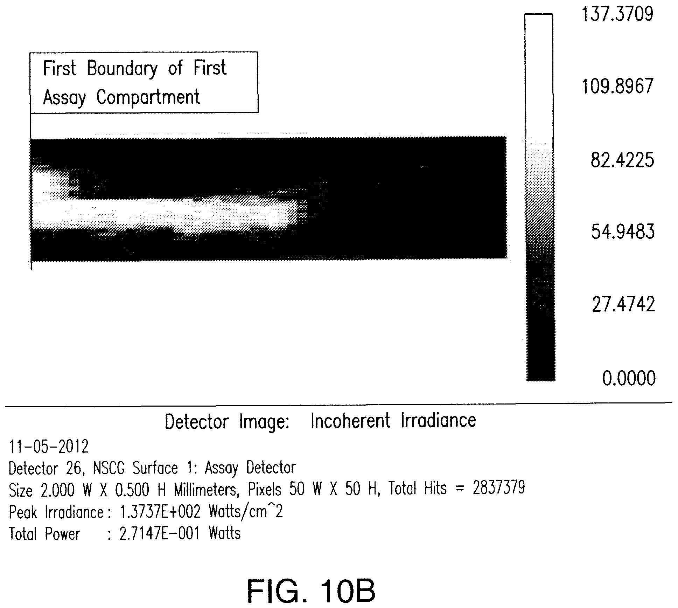

Figures 8-10A show the fluorescence intensity distribution inside the assay compartment 420 for each of the aforementioned designs. Clearly, while, with the straight cut fiber, the fluorescence emission zone extends out to the membrane, the CPC concentrates the excitation and the fluorescence emission in the center of the assay compartment. Figure 10B shows the fluorescence intensity distribution inside the assay compartments for the side cut cavities shown in Figures 7C and 7D.

It is noted that the CPC configuration applied to the fiber sensor in the above description is illustrative, and other geometries, e.g., a CPC with a rectangular cross sectional area, as well as other imaging or non-imaging geometries that also change the numerical aperture of the fiber tip may also be applied.

The disclosure is also directed to improved optical systems for interrogating fluorophore-labeled assays contained within optical sensors and, in particular, fiber optical sensors of the types described above.

As noted previously, in typical interrogation systems (e.g., epifluorescence microscopy), fluorescence applications commonly operate with fairly intense excitation of the fluorophore in the absorption band of the fluorophore and detection of the weak fluorescence emitted by the fluorophore. In such applications, the dichroic beam splitter serves as a crude filtering of the light, whereas the heavy filtering occurs in the excitation filter and the emission filter, which are used in transmission mode and depend on $10^6$ times attenuation of wavelengths outside the pass band in a single pass. Such filters are normally based on dielectric multilayer filters consisting of a substrate with optical coatings on both sides consisting of up to 100 layers with alternating refractive indices. In the pass band, the filters have up to 99% transmittance, which is most probably caused by reflection losses in the air-coating transition on both sides of the filter in spite of the fact that filter stacks on both sides of the substrate generally include an anti-reflective coating.

In accordance with the present disclosure, an optical system may be used for interrogating a fluorophore-labeled assay either with, or without, an internal reference. The optical system is based on a filter substrate with one or multiple optical coatings separated in location on the surfaces of the substrate. The coatings may be dielectrical multilayer coatings forming short wave pass, longwave pass, band pass filters, and anti-reflection coatings. Furthermore, coatings may be metallic reflective coatings.

More specifically, exemplary optical systems of the present disclosure utilize the fact that a dielectrical multilayer filter reflects what is not transmitted. Therefore, a filter in accordance with the present disclosure may include a first coating on the filter substrate which transmits a certain wavelength range of the excitation, but reflects wavelengths outside the pass band. This enables the emitted fluorescence to be reflected once or multiple times by the coating, while allowing reflected excitation light to be transmitted out of the optical system. The filtered fluorescence then exits the filter substrate when it reaches a part of the substrate that is not coated with the first coating, and is picked up by one or more detectors.

Subsequent filtering may also be achieved by applying a second coating which transmits a desired wavelength range originating from a first fluorophore, but reflects wavelengths outside the transmission band - in particular, remains of the excitation light. In this way, the desired wavelength range will be transmitted out of the substrate where it may be picked up by one or more suitable detectors. In addition, anti-reflective coatings may be applied to areas of the substrate where light is coupled into, or out of, the substrate to reduce reflection losses.

In an intensity interrogation configuration, where both a first fluorophore and a second fluorophore are used, the above-mentioned second coating may transmit the desired wavelength range associated with the first fluorophore and reflect wavelengths outside the transmission band - in particular remains of the excitation light and fluorescence related to the second fluorophore. The filtered fluorescence originating from the second fluorophore then exits the filter substrate when it reaches a part of the substrate that is not coated with the first or second coating, such that it can be picked up by a suitable detector(s). Subsequent filtering of fluorescence originating from the second fluorophore may be achieved by applying a second coating which transmits the desired wavelength range associated with the second fluorophore and reflects wavelengths outside the transmission band - in particular, remains of the excitation light. In this way, the desired wavelength range may be transmitted out of the substrate where it may be picked up by a suitable detector(s). In other examples, the system may be expanded to include multiple light sources and/or multiple wavelength ranges to be detected.

Implementation of the above-described filter configurations requires imaging optical elements such as lenses, mirrors or diffractive optical elements to focus, pick up, and collimate light. Furthermore, apertures and light traps may be required to control light path and to absorb undesired wavelengths that are transmitted out of the filter substrate, such as, e.g., the excitation light to be blocked. In this example, the above-mentioned optical elements and light traps are shown with an air gap between the elements and the (glass) substrate. However, the optical elements and light traps may also be formed in an optically transparent material with mirror coating and absorbing coating on the surface facing away from the filter substrate. Such a configuration is generally more advantageous, as the optical elements and light traps are better index matched to the filter substrate/filter coating than is the case when an air gap is present.

The above-mentioned elements may be individually aligned and fitted onto both sides of the coated filter substrate as discrete units. Similarly, one or more light sources and one or more detectors may be aligned and fitted onto the filter substrate as packaged units, as raw dies laminated directly onto the coated filter substrate, or mounted as raw dies onto a printed circuit board and mounted as a unit onto the coated filter substrate.

Suitable light sources may include, e.g., light emitting diodes (LEDs) and laser diodes, and suitable detectors may include, e.g., photodiodes, avalanche photodiodes, silicon photomultipliers, photomultipliers, and phototransistors. In addition, the assembled optical system may be coated or placed in an enclosure to block out ambient light.

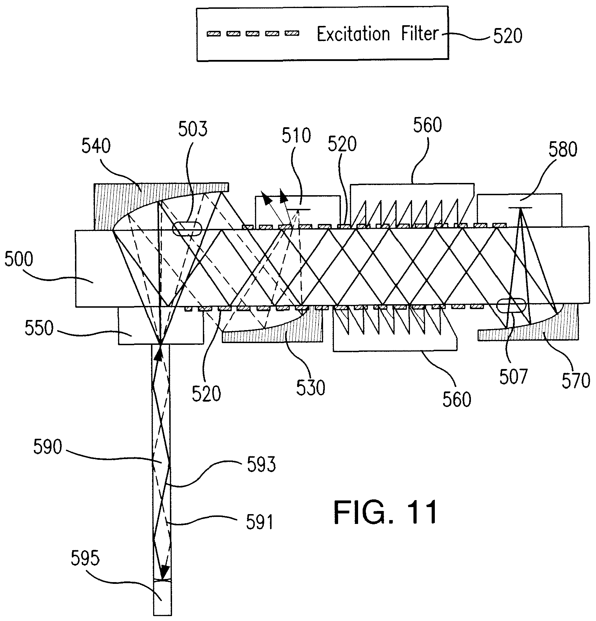

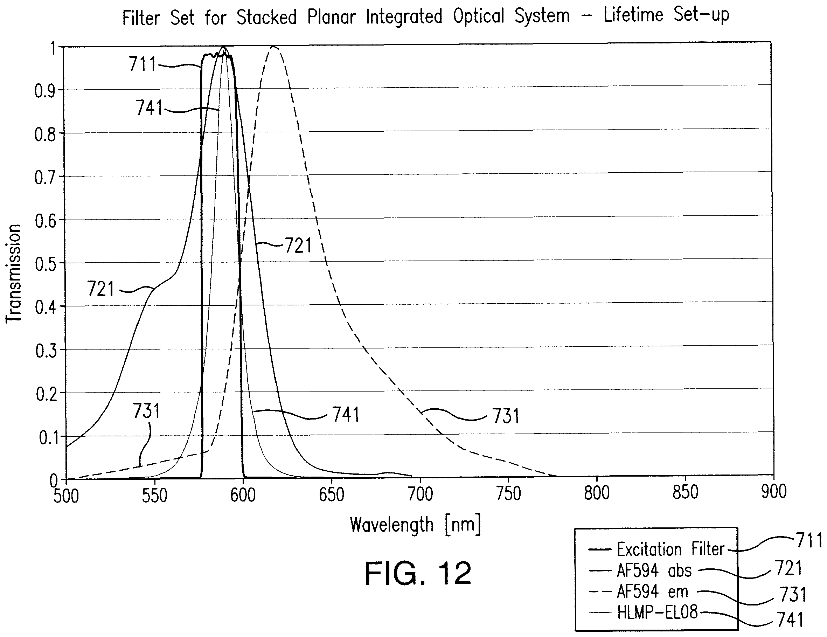

A stacked planar integrated optical system (SPIOS) for interrogating a single fluorophore is shown in Figure 11. With reference to Figures 11 and 12, a LED 510 emits light into a filter substrate 500 with a wavelength range overlapping the absorption spectrum of the fluorophore to be interrogated. The LED output is limited to a certain wavelength range by an excitation filter 520 before entering the filter substrate 500. The filtered excitation light exits the filter substrate 500 through an identical excitation filter on the opposite side of the filter substrate and is collimated by a first mirror 530. The collimated excitation light passes through the filter substrate to reach a second mirror 540 and is thereby focused onto a sensor 590 through an optical window 550. It is noted that, in this example, the sensor 590 is a fiber optical sensor--including an assay in an assay compartment 595--of the types described above in connection with Figures 5-10.

Traveling through the sensor 590, the excitation light 591 reaches the assay compartment 595, where it excites the fluorophore in the assay such that the fluorophore emits fluorescence 593. Furthermore, excitation light is reflected and back scattered from the optical window and sensor. The fluorescence 593 and reflected/back scattered excitation light are picked up and collimated by the second mirror 540 and enter the filter substrate 500 through an uncoated or anti-reflection coated area 503 on the filter substrate. The emitted fluorescence is reflected between the two coated surfaces (i.e., the excitation filters 520) while the excitation light is transmitted through the coatings 520 and absorbed by the light traps 560. The filtered fluorescence exits the filter substrate 500 where the coated area ends 507 and is focused by a third mirror 570 onto a detector 580 through an uncoated, or anti-reflection coated, region of the filter substrate.

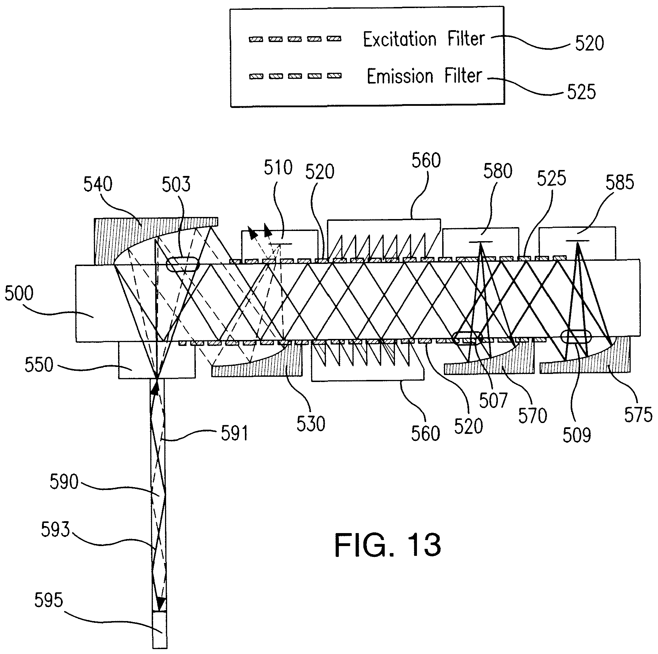

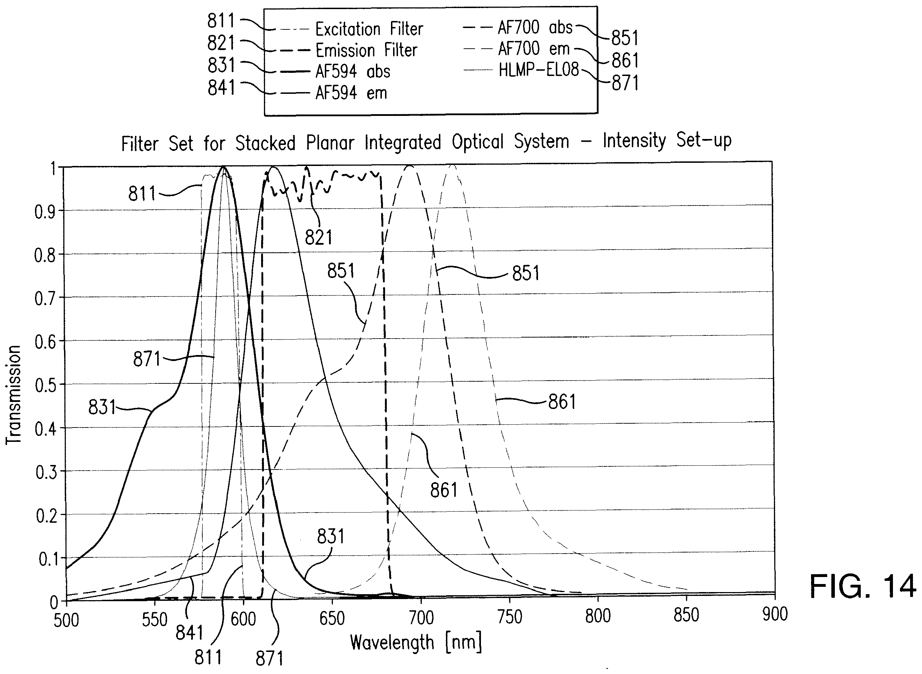

In an alternative example, a stacked planar integrated optical system (SPIOS) for interrogating a sensor with an assay fluorophore and a reference fluorophore is shown in Figure 13. With reference to Figures 13 and 14, a LED 510 emits light into a filter substrate 500 with a wavelength range overlapping the absorption spectrum of the two fluorophores to be interrogated. The LED output is limited to a certain wavelength range by an excitation filter 520 before entering the filter substrate 500. The filtered excitation light exits the filter substrate 500 through an identical excitation filter on the opposite side of the filter substrate and is collimated by a first mirror 530. The collimated excitation light passes through the filter substrate to reach a second mirror 540 and is thereby focused onto a sensor 590 through an optical window 550. It is noted that, in this example, the sensor 590 is a fiber optical sensor--including an assay in an assay compartment 595--of the types described above in connection with Figures 5-10.

Traveling through the sensor 590, the excitation light 591 reaches the assay compartment 595, where it excites the fluorophore in the assay such that the fluorophore emits fluorescence 593. Furthermore, excitation light is reflected and back scattered from the optical window and sensor. The fluorescence 593 and reflected/back scattered excitation light are picked up and collimated by the second mirror 540 to enter the filter substrate 500 through an uncoated or anti-reflection coated area 503 on the filter substrate. The emitted fluorescence is reflected between the two excitation filter coatings 520 while the excitation light is transmitted through the coatings and absorbed by the light traps 560.

The filtered fluorescence exits the filter substrate 500 where the excitation filter coatings end 507, and an emission filter 525 transmits a wavelength range relating to a first fluorophore (the assay fluorophore). The filtered fluorescence from the first fluorophore is focused by a third mirror 570 onto a first detector 580, while the fluorescence associated with the second fluorophore (the reference fluorophore) is reflected between the emission filter 525 on the two sides of the filter substrate 500. The filtered fluorescence from the second fluorophore exits the filter substrate 500 where the coated area ends 509 or is replaced with an anti-reflection coating, and is focused by a fourth mirror 575 onto a second detector 585 through an uncoated, or anti-reflection coated, region of the filter substrate.

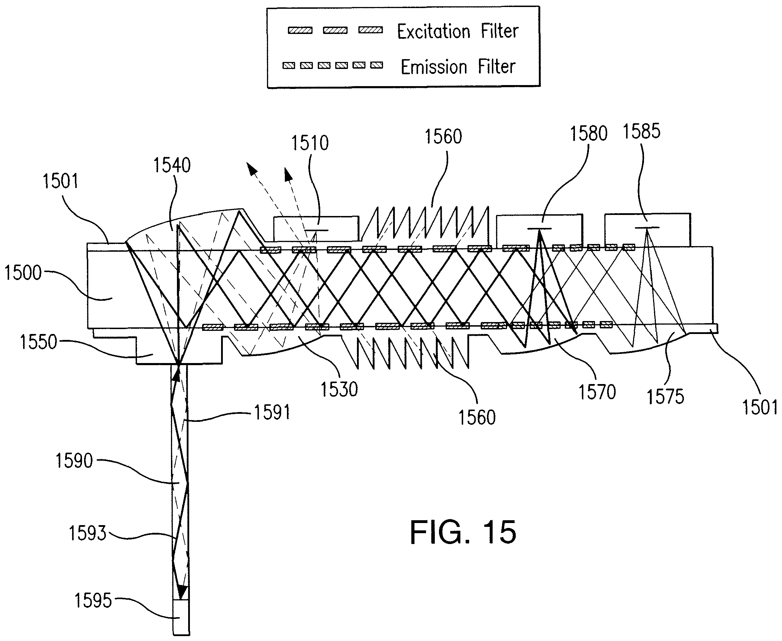

As noted previously, the optical elements, apertures, and light traps of the SPIOS for interrogating a sensor with an assay fluorophore and a reference fluorophore may be disposed with an air gap between the elements and the (glass) substrate. See Figure 13. However, the optical elements and light traps may also be formed in an optically transparent material with mirror coating and absorbing coating on the surface facing away from the filter substrate. The latter configuration is generally more advantageous, as the optical elements and light traps are better index matched to the filter substrate/filter coating than is the case when an air gap is present. An example of such a configuration is shown in Figure 15, where connecting material 1501 is provided between the optical components.

It is noted that the light emitting area of a LED chip has an area, which is comparable to a 500 $\mu$m multimode fiber. Furthermore, the LED chip emits in a large angular space. For a SPIOS equipped with a LED interrogating a fiber sensor, the fluorescence output gradually becomes limited by the ability to focus light from the LED onto the fiber when the fiber diameter is reduced. Also the positioning of the proximal end of the fiber relative to the optical system becomes more critical.

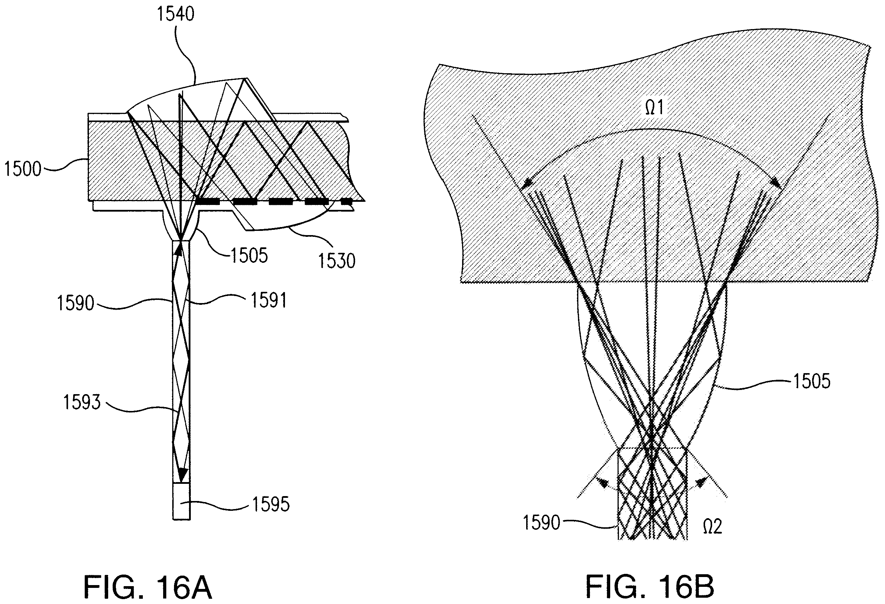

As shown in Figures 16A and 16B, the ability to couple light from the LED into the fiber sensor may be enhanced by placing a compound parabolic concentrator (CPC) 1505 in the interface between the SPIOS and the fiber sensor 1590 if the numerical aperture of the SPIOS in relation to the fiber is smaller than the numerical aperture of the fiber itself. The CPC may then be used to match the numerical aperture of the fiber and thereby reduce the spot size of the focused excitation light originating from the LED. This, in turn, enables more light to be coupled from the LED into the fiber.

The CPC may be an integral part of the SPIOS, as shown, e.g., in Figure 16A. However, the CPC may also be formed on the proximal end of the fiber, whereby the area to be targeted by the SPIOS is increased, thus reducing the requirements on the positioning of the fiber (with CPC at the proximal end) relative to the SPIOS.

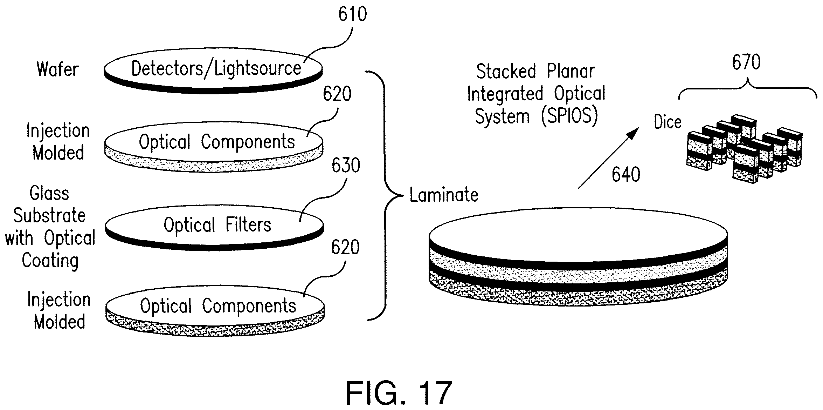

In yet another example shown in Figure 17, the optical interrogating system may be designed to be manufactured as a wafer-scale stacked planar integrated optical system, or wafer-scale SPIOS (also referred to as a "Wafer Scale Optical System" or a "Wafer Level Optical System"). As shown in Figure 17, the SPIOS includes various layers that are stacked and aligned. In the wafer layer 610, one or more light sources (e.g., LEDs and photodiodes) and detectors may be laid out on a wafer. Alternatively, they may be naked chips (e.g., sold by Avago Technologies or Hamamatsu), which are individually aligned and laminated onto the SPIOS units.

One or more optical layers 620 may include mirrors and absorbers laid out on a wafer-sized injection molded disk. Mold inserts defining optical surfaces are made by a diamond turning/milling company (e.g., Kaleido Technology in Denmark). Gold or protected silver is applied to mirror surfaces, e.g., by sputtering, while any absorbers are masked off during the process.

The optical filter layer 630 includes a wafer-sized glass substrate with optional coatings. Specifically, multilayer optical coatings may be applied on both sides of the glass substrate using ion-assisted sputtering to form durable coatings. The technique is similar to that used in manufacturing fluorescence filters by, e.g., Semrock in the United States and Delta in Denmark.

As shown in Figure 17, in one example, a wafer layer 610 may be followed by an optical layer 620, an optical filter layer 630, and another optical layer 620. The entire stack is then thoroughly aligned and laminated, e.g., by gluing, and the connections are bonded onto the chips. The stack is then diced 640 using, e.g., a diamond saw to form multiple assembled SPIOS units 670.

The above-described system may be made small and is suitable for large-scale production. The system may be used for interrogating a sensor in a light scattering environment, such as a sensor implanted into the skin, as well as a fiber sensor. Coating or packaging may be used to block out ambient light.

The presently disclosed embodiments are therefore to be considered in all respects as illustrative and not restrictive, the scope of the invention being indicated by the appended claims, and all changes which come within the meaning and range of equivalency of the claims are therefore intended to be embraced therein.

**Claims**

1. An optical glucose sensor (400) comprising:

an optical fiber (410) having a proximal end and an opposing distal end (412) ;

a glucose-permeable membrane (430) having a hollow interior, an open proximal end, and a closed distal end, wherein the membrane's proximal end is coupled to the optical fiber's distal end so as to define a compartment (420) in said hollow interior between the optical fiber's distal end and the membrane's distal end; and

a competitive glucose binding affinity assay disposed in said compartment, the assay including a glucose receptor and a glucose analog;

**characterized in that**

the glucose receptor is Mannan Binding Lectin (MBL) labeled with Alexa Fluor 594 (AF594);

the glucose analog is dextran labeled with hexamethoxy crystalviolet-1 (HMCV 1); and the assay further includes a macro molecule labeled with Alexa Fluor 700 (AF700) as a reference fluorophore.

2. The optical glucose sensor of claim 1, wherein the compartment (420) is configured to be placed within a user's tissue.

3. The optical glucose sensor of claim 2, wherein said proximal end of the optical fiber (410) is configured to be external to the user's body.

4. The optical glucose sensor of claim 3, wherein the proximal end of the optical fiber (410) is configured to be optically coupled to an assay interrogating system.

5. The optical glucose sensor of claim 2, wherein the proximal end of the optical fiber (410) is configured to be optically coupled to an assay interrogating system.

6. The optical glucose sensor of claim 5, wherein the interrogating system is an optical interrogating system.

7. The optical glucose sensor of claim 1, wherein said membrane (430) is tube-shaped.

8. The optical glucose sensor of claim 1, wherein the membrane's (430) open proximal end is sealably fitted over the optical fiber's distal end (412).

9. The optical glucose sensor of claim 1, wherein the distal end (412) of the optical fiber (410) is straight cut and polished.

10. The optical glucose sensor of claim 1, wherein the distal end (412) of the optical fiber (410) is in direct contact with the assay.


**Patentansprüche**

1. Optischer Glukosesensor (400), umfassend:

eine optische Faser (410),

die ein proximales Ende und ein gegenüberliegendes distales Ende (412) aufweist;

eine glukosedurchlässige Membran (430), die einen hohlen Innenraum, ein offenes proximales Ende und ein geschlossenes distales Ende aufweist, wobei das proximale Ende der Membran mit dem distalen Ende der optischen Faser gekoppelt ist, um in dem hohlen Innenraum zwischen dem distalen Ende der optischen Faser und dem distalen Ende der Membran einen Raum (420) zu definieren; und

einen kompetitiven Glukose-Bindungsaffinitätsassay, der in dem Raum angeordnet ist, wobei der Assay einen Glukoserezeptor und ein Glukoseanalogon einschließt;

**dadurch gekennzeichnet, dass**

der Glukoserezeptor Mannan bindendes Lektin (MBL) ist, das mit Alexa Fluor 594 (AF594) markiert ist;

das Glukoseanalogon Dextran ist, das mit Hexamethoxykristallviolett-I (HMCV 1) markiert ist; und

der Assay ferner ein Makromolekül einschließt, das mit Alexa Fluor 700 (AF700) als Referenzfluorophor markiert ist.

2. Optischer Glukosesensor nach Anspruch 1, wobei der Raum (420) konfiguriert ist, um innerhalb des Gewebes eines Benutzers platziert zu werden.

3. Optischer Glukosesensor nach Anspruch 2, wobei das proximale Ende der optischen Faser (410) konfiguriert ist, um außerhalb des Körpers des Benutzers vorzuliegen.

**4.** Optischer Glukosesensor nach Anspruch 3, wobei das proximale Ende der optischen Faser (410) konfiguriert ist, um optisch mit einem Assay-Abfragesystem gekoppelt zu sein.

**5.** Optischer Glukosesensor nach Anspruch 2, wobei das proximale Ende der optischen Faser (410) konfiguriert ist, um optisch mit einem Assay-Abfragesystem gekoppelt zu sein.

**6.** Optischer Glukosesensor nach Anspruch 5, wobei das Abfragesystem ein optisches Abfragesystem ist.

**7.** Optischer Glukosesensor nach Anspruch 1, wobei die Membran (430) röhrenförmig ist.

**8.** Optischer Glukosesensor nach Anspruch 1, wobei das offene proximale Ende der Membran (430) abdichtbar über dem distalen Ende der optischen Faser (412) angebracht ist.

**9.** Optischer Glukosesensor nach Anspruch 1, wobei das distale Ende (412) der optischen Faser (410) gerade geschnitten und poliert ist.

**10.** Optischer Glukosesensor nach Anspruch 1, wobei das distale Ende (412) der optischen Faser (410) in direktem Kontakt mit dem Assay ist.

**Revendications**

**1.** Capteur optique de glucose (400) comprenant :

une fibre optique (410)
ayant une extrémité proximale et une extrémité distale opposée (412) ;
une membrane perméable au glucose (430) ayant un intérieur creux, une extrémité proximale ouverte et une extrémité distale fermée, dans lequel l'extrémité proximale de la membrane est couplée à l'extrémité distale de la fibre optique de façon à définir un compartiment (420) dans ledit intérieur creux entre l'extrémité distale de la fibre optique et l'extrémité distale de la membrane ; et
un dosage compétitif d'affinité de liaison de glucose disposé dans ledit compartiment, le dosage incluant un récepteur de glucose et un analogue de glucose ;
**caractérisé en ce que**
le récepteur de glucose est de la lectine mannose-spécifique (MBL) marquée avec Alexa Fluor 594 (AF594) ;
l'analogue de glucose est du dextrane marqué avec de l'hexaméthoxy cristal-violet-I (HMCV 1) ; et
le dosage inclut en outre une macromolécule marquée avec Alexa Fluor 700 (AF700) en tant que fluorophore de référence.

**2.** Capteur optique de glucose selon la revendication 1, dans lequel le compartiment (420) est configuré pour être placé au sein d'un tissu d'un utilisateur.

**3.** Capteur optique de glucose selon la revendication 2, dans lequel ladite extrémité proximale de la fibre optique (410) est configurée pour être externe au corps de l'utilisateur.

**4.** Capteur optique de glucose selon la revendication 3, dans lequel l'extrémité proximale de la fibre optique (410) est configurée pour être couplée optiquement à un système d'interrogation de dosage.

**5.** Capteur optique de glucose selon la revendication 2, dans lequel l'extrémité proximale de la fibre optique (410) est configurée pour être couplée optiquement à un système d'interrogation de dosage.

**6.** Capteur optique de glucose selon la revendication 5, dans lequel le système d'interrogation est un système d'interrogation optique.

**7.** Capteur optique de glucose selon la revendication 1, dans lequel ladite membrane (430) est en forme de tube.

**8.** Capteur optique de glucose selon la revendication 1, dans lequel l'extrémité proximale ouverte de la membrane (430) est montée de manière étanche par-dessus l'extrémité distale de la fibre optique (412).

9. Capteur optique de glucose selon la revendication 1, dans lequel l'extrémité distale (412) de la fibre optique (410) est coupée droite et polie.

10. Capteur optique de glucose selon la revendication 1, dans lequel l'extrémité distale (412) de la fibre optique (410) est en contact direct avec le dosage.

AF594 Labeled MBL

HMCV1 Labeled Dextran

○ Glucose

FIG. 1

FIG. 2

FIG. 3

FIG. 4A

EP 2 753 918 B1

FIG. 4B

FIG. 5A

FIG. 5B

FIG. 6A

FIG. 6B

FIG. 7A

FIG. 7B

FIG. 7C

FIG. 7D

Detector Image:   Incoherent Irradiance

14-12-2011
Detector 17, NSCG Surface 1: Assay Detector
Size 1.298 W X 0.498 H Millimeters, Pixels 50 W X 50 H, Total Hits = 64189
Peak Irradiance: 3.4517E+001 Watts/cm^2
Total Power    : 2.4732E-002 Watts

FIG. 8

EP 2 753 918 B1

Fiber Tip

108.7364

86.9891

65.2418

43.4945

21.7473

0.0000

Detector Image:   Incoherent Irradiance

14-12-2011
Detector 17, NSCG Surface 1: Assay Detector
Size 1.298 W X 0.498 H Millimeters, Pixels 50 W X 50 H, Total Hits = 100530
Peak Irradiance: 1.0874E+002 Watts/cm^2
Total Power    : 2.9066E-002 Watts

FIG. 9

EP 2 753 918 B1

Detector Image:   Incoherent Irradiance

14-12-2011
Detector 17, NSCG Surface 1: Assay Detector
Size 1.298 W X 0.498 H Millimeters, Pixels 50 W X 50 H, Total Hits = 64189
Peak Irradiance : 3.4517E+001 Watts/cm^2
Total Power    : 2.4732E-002 Watts

FIG. 10A

EP 2 753 918 B1

Detector Image:   Incoherent Irradiance

11-05-2012
Detector 26, NSCG Surface 1: Assay Detector
Size 2.000 W X 0.500 H Millimeters, Pixels 50 W X 50 H, Total Hits = 2837379
Peak Irradiance: 1.3737E+002 Watts/cm^2
Total Power    : 2.7147E-001 Watts

FIG. 10B

Excitation Filter — 520

FIG. 11

Filter Set for Stacked Planar Integrated Optical System – Lifetime Set-up

FIG. 12

| | |
|---|---|
| —— Excitation Filter | 711 |
| —— AF594 abs | 721 |
| - - - AF594 em | 731 |
| —— HLMP-EL08 | 741 |

EP 2 753 918 B1

FIG. 13

Filter Set for Stacked Planar Integrated Optical System – Intensity Set-up

FIG. 14

FIG. 15

EP 2 753 918 B1

FIG. 16A

FIG. 16B

EP 2 753 918 B1

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2003171666 A

### Non-patent literature cited in the description

- **R. BALLERSTADT et al.** Fiber-Coupled Fluorescence Affinity Sensor for 3-Day in Vivo Glucose Sensing. *Journal of Diabetes Science and Technology,* May 2007, vol. 1 (3), 384-393

- **J. K. NIELSEN et al.** Clinical Evaluation of a Transcutaneous Interrogated Fluorescence Lifetime-Based Microsensor for Continuous Glucose Reading. *Journal of Diabetes Science and Technology,* January 2009, vol. 3 (1), 98-109